# EUROPÄISCHE PATENTANMELDUNG

(11) **EP 4 529 897 A1**
(43) Veröffentlichungstag der Anmeldung: **02.04.2025**
(21) Anmeldenummer: 23200861.5
(22) Anmeldetag: 29.09.2023
(51) Int. Cl.: A61F 2/915

(54) **IMPLANTAT ZUR WIEDERHERSTELLUNG DER DURCHGÄNGIGKEIT UND FUNKTIONALITÄT DES EILEITERS IM REPRODUKTIONSGESCHEHEN**

(71) Anmelder: Institut für Implantattechnologie und Biomaterialien e.V., 18119 Rostock-Warnemünde (DE); Universitätsmedizin Greifswald, 17457 Greifswald (DE)
(72) Erfinder: Schmitz, Klaus-Peter, 18119 Rostock (DE); Bock, Andrea, 18119 Rostock-Warnemünde (DE); Siewert, Stefan, 18055 Rostock (DE); Dierke, Ariane, 18249 Penzin (DE); Stiehm, Michael, 18146 Markgrafenheide (DE); Zygmunt, Marek, 17475 Greifswald (DE); Muzzio, Damián Oscar, 17475 Greifswald (DE); Normann, Nicole, 17475 Greifswald (DE); Alwafai, Zaher, 17475 Greifswald (DE); Spring, Paula, 17475 Greifswald (DE)
(74) Vertreter: Heinemeyer, Karsten

(57) **Zusammenfassung**

Beschrieben wird ein Implantat (3) zur Wiederherstellung der Durchgängigkeit und Funktionalität des Eileiters im Reproduktionsgeschehen. Das Implantat (3) verfügt über ein Stützelement (17) mit einer rohrförmigen, ein Innenvolumen (20) des Stützelements (17) zumindest teilweise begrenzenden und einen zumindest annähernd kreisförmigen Querschnitt aufweisenden Außenwand (18), wobei in der Außenwand (18) Öffnungen (19) vorgesehen sind, die derart ausgebildet sind, dass zumindest zeitweise nach Implantation des Stützelements (17) Falten einer Schleimhaut des Eileiters wenigstens bereichsweise durch die Öffnungen (19) in das Innenvolumen (20) hindurchwachsen und/oder hindurchragen.

Das beschriebene Implantat (3) zeichnet sich dadurch aus, dass eine Steifigkeit des Stützelements (17) in Längsrichtung des Stützelements variiert.

## Beschreibung

Die Erfindung Implantat zur Wiederherstellung der Durchgängigkeit und Funktionalität des Eileiters im Reproduktionsgeschehen. Das beschriebene Implantat verfügt über ein Stützelement mit einer rohrförmigen, ein Innenvolumen des Stützelements zumindest teilweise begrenzenden und einen zumindest annähernd kreisförmigen Querschnitt aufweisenden Außenwand. Hierbei sind in der Außenwand Öffnungen vorgesehen, die derart ausgebildet sind, dass zumindest zeitweise nach Implantation des Stützelementes in einen Eileiter Falten einer Schleimhaut des Eileiters wenigstens bereichsweise durch die Öffnungen in das Innenvolumen hindurchwachsen und/oder hindurch tragen. Durch das erfindungsgemäße Implantat wird gezielt eine Verbesserung der Passierbarkeit oder dauerhafte Gewährleistung des Durchlässigkeitsvermögens für Spermien und/oder befruchtete Eizellen durch den Eileiter in die Gebärmutter gewährleistet.

Bis zu 15% der Paare im sogenannten reproduktiven Alter leiden unter unerfülltem Kinderwunsch. Sofern Paare keine Kinder bekommen können, sind oftmals Verwachsungen im Eileiter der Frau eine der möglichen Ursachen für den unerfüllten Kinderwunsch. Eizellen und Spermien können sich dann nur sehr schwer durch den Eileiter bewegen und eine Schwangerschaft bleibt oft aus.

Zumeist können die Spermien den Ort der Befruchtung nicht erreichen, da der Verschluss im proximalen Bereich liegt. Tritt trotzdem eine Befruchtung ein, kann es zu einer Eileiterschwangerschaft kommen, da die befruchtete Zelle die Engstelle nicht passieren kann.

Die Eileiter sind 10 bis ca. 12 cm lange muskulöse Schläuche, die den Eierstock mit der Gebärmutter verbinden. Ihre Funktion ist fundamental für die Befruchtung, da die Keimzellen vor der Befruchtung im Eileiter transportiert werden, die Eizelle vom Eierstock uteruswärts gerichtet, die Samenzellen vom Uterus aus aufwärts Richtung Eierstock. Die Eileiter dürfen somit nicht verschlossen sein. Außerdem müssen die Eileiter beweglich sein, um nach dem Eisprung die Eizelle aufnehmen und durch muskuläre Kontraktionen in Richtung der Samenzellen bewegen zu können.

Die menschlichen Eileiter sind als paarig angelegte muskuläre Hohlorgane im kleinen Becken (Pelvis minor) des weiblichen Körpers lokalisiert und werden vom Bauchfell (Peritoneum) umgeben. Der proximale Bereich des Eileiters, der Isthmus, erstreckt sich über eine Länge von ca. 3 bis 4 cm und weist neben einem geringen Gefäßlumendurchmesser von 0,1 bis 1 mm eine stark ausgeprägte, 2 bis 3 mm dicke Wandmuskulatur auf.

Außerdem bildet die sogenannte Tubenschleimhaut eine Vielzahl von Längs-, Sekundärsowie Tertiärfalten aus und setzt sich vorwiegend aus Drüsenzellen und Flimmerzellen, den Kinozilien, zusammen. Hierbei unterstützt der Flimmerschlag der Kinozilien den Eizellentransport und erzeugt einen uteruswärts gerichteten Sekretstrom, der die Spermien mitführt.

Der Verschluss der Eileiter ist eine der Hauptgründe für die feminine Infertilität, da der Verbindungskanal zwischen Eierstock und Gebärmutter unterbrochen ist. Die Ursachen für einen Eileiterverschluss sind unterschiedlich.

Um einen derartigen Eileiterverschluss oder eine Verengung zu behandeln, werden in vielen Fällen, sofern dies überhaupt möglich ist, chirurgische Eingriffe oder eine Kathetisierung vorgenommen. Es hat sich allerdings mittlerweile gezeigt, dass diese Eingriffe zur Herstellung der Eileiterdurchlässigkeit nicht nur risikobehaftet, sondern auch in Bezug auf die Schwangerschaftsraten nur mäßig erfolgreich sind. Ferner kann es zu postoperativen Komplikationen, Schmerzen und einer Beeinträchtigung der Funktionalität durch die Verkürzung des Eileiters und der Bildung von Narbengewebe kommen. Demgegenüber zeichnet sich die Katheterisierung von Engstellen durch befriedigende Schwangerschaftserfolgsquoten in den ersten Monaten nach erfolgter Behandlung aus. Allerdings sind die Wiederverschlussraten in der darauffolgenden Zeit vergleichsweise hoch.

Aus diesen Gründen wird bei einem diagnostizierten Eileiterverschluss oftmals empfohlen, auf die Methoden der assistierten Reproduktion, wie etwa eine In-Vitro-Fertilisation (IVF), zurückzugreifen. Auch diese Verfahren weisen allerdings Nachteile auf, da sie mit der Einnahme von Hormonen in nicht unerheblichen Dosen und den damit verbundenen Nebenwirkungen einhergehen sowie die Schwangerschaftserfolsquote nach einem Zyklus nur unzureichend sind. Darüber hinaus spielen ethische und religiöse Aspekte oftmals eine Schlüsselrolle im Zusammenhang mit einer IVF. Nicht zuletzt ist die IVF mit einem erheblichen finanziellen Aufwand verbunden.

Neben den zuvor beschriebenen Verfahren zur Behandlung verschlossener oder verengter Eileiter sind ferner generell Stützelemente bekannt, die teilweise bei der Behandlung von Eileiterverletzungen oder Eileitererkrankungen zum Einsatz kommen. So ist etwa aus der DE 602 22 565 T2 ein spiralförmiger Stent aus biokompatiblem Metall bekannt, der generell geeignet sein soll, Körperhöhlen offenzuhalten. Bei Einsatz eines derartigen Stents im Bereich eines Eileiters besteht jedoch die Gefahr, dass es zu einer chronischen Entzündung im proximalen Bereich des Eileiters kommt. sodass das Risiko eines Verschlusses des Eileiters und einer daraus resultierenden Sterilität besteht.

Im Weiteren sind Implantate, wie sie etwa in der CN 113017948 A beschrieben werden, bekannt, die vor oder nach einer Operation als Medikamentendepot im Bereich des Eileiters eingesetzt werden.

Keine der zuvor beschriebenen technischen Lösungen gewährleistet allerdings die nachhaltige Öffnung eines Eileiters oder die Beseitigung einer Engstelle im Eileiter, sodass komplikationsfrei ein Kanal im Eileiter geschaffen wird, der längerfristig für Spermien und/oder befruchtete Eizellen passierfähig ist.

Ausgehend von den aus dem Stand der Technik bekannten technischen Lösungen sowie den zuvor beschriebenen Problemen, die aus einer Verengung oder einem Verschluss eines Eileiters herrühren können, liegt der Erfindung die Aufgabe zu Grunde, mit vergleichsweise einfachen Mitteln die Passierbarkeit eines zuvor verschlossenen oder zumindest abschnittsweise verengten Eileiters sicherzustellen. Wesentlich hierbei ist, dass lediglich ein kleiner Eingriff erforderlich sein soll, um auch über eine vergleichsweise lange Zeitspanne von mehreren Wochen, Monaten oder sogar Jahren die Passierbarkeit eines Eileiters für Spermien und/oder befruchtete Eizellen und somit eine natürliche Schwangerschaft zu ermöglichen. Die anzugebende technische Lösung sollte für eine Patientin mit keinen Nebenwirkungen und Risiken verbunden und für einen Behandler einfach und intuitiv umsetzbar sein. Im Weiteren sollte die Erfindung problemlos bei Patientinnen mit unterschiedlicher Anatomie und Größe, insbesondere bei unterschiedlich geformten Eileitern einsetzbar oder auf einfache Weise adaptierbar sein. Darüber hinaus soll die natürliche Funktion des Eileiters nicht oder nur minimal beeinträchtigt werden, wobei es von besonderer Bedeutung ist, dass die Funktionstüchtigkeit der Eileiter als hormonelle Klappe und der Tubenschleimhaut des Eileiters auch nach erfolgter Implantation des erfindungsgemäßen Implantats gewährleistet ist, um so eine möglichst komplikationsfreie Bewegung von Spermien und/oder befruchteten Eizellen durch einen Eileiter sicherzustellen.

Die zuvor angegebene Aufgabe wird mithilfe eines Implantats nach Anspruch 1 gelöst. Vorteilhafte Ausführungsformen der Erfindung sind in den abhängigen Ansprüchen angegeben und werden in der folgenden Beschreibung unter teilweiser Bezugnahme auf Figuren näher erläutert.

Die Erfindung betrifft ein Implantat zur Wiederherstellung der Durchgängigkeit und Funktionalität des Eileiters im Reproduktionsgeschehen, wobei das Implantat über ein Stützelement mit einem rohrförmigen, ein Innenvolumen des Stützelements zumindest teilweise begrenzenden und einen zumindest annähernd kreisförmigen Querschnitt aufweisenden Außenwand verfügt. Hierbei sind in der Außenwand Öffnungen vorgesehen, die derart ausgebildet sind, dass zumindest zeitweise nach Implantation des Stützelementes Falten einer Schleimhaut des Eileiters wenigstens bereichsweise durch die Öffnungen in das Innenvolumen, das von der Außenwand wenigstens bereichsweise begrenzt ist, hindurchwachsen und oder hindurchragen. Es wird somit ein Implantat mit einem rohrförmigen Stützelement bereitgestellt, das an seinen beiden in Längsrichtung gegenüberliegend angeordneten Enden Ein- bzw. Auslässe aufweist und über eine offenporig ausgestaltete Außenwand verfügt. Das erfindungsgemäße Implantat zeichnet sich dadurch aus, dass eine Steifigkeit des Stützelements in Längsrichtung des Stützelementes variiert. Die erfindungsgemäße Lösung beruht somit auf der Idee, dass das Stützelement, das über eine Mehrzahl von Öffnungen in der Außenwand verfügt, in Längsrichtung eine sich ändernde Steifigkeit aufweist. Eine derartige Gestaltung führt dazu, dass die Steifigkeit im Bereich eines ersten Endes des Stützelementes größer ist als eine durchschnittliche Steifigkeit des Stützelementes und im Bereich des gegenüberliegenden, zweiten Endes die Steifigkeit niedriger als die mittlere Steifigkeit ist. Gleichzeitig ist das Stützelement derart ausgeführt, dass sich das Implantat aufgrund der radialen Steifigkeit des Stützelementes im Isthmus des Eileiters fest verankern lässt, sodass dieser dauerhaft dilatiert bzw. offengehalten und dennoch die Klappenfunktion der Eileiter nicht beeinträchtigt wird., Dies wird auf vorteilhafte Weise erreicht, indem sich das Implantat bei Muskelkontraktion im Querschnitt auf sein Minimum komprimieren lässt und bei anschließender Relaxation selbstständig wieder auf sein Standardmaß expandiert. Mithilfe des erfindungsgemäßen Implantats lässt sich der Eileiter, insbesondere eine Engstelle des Eileiters Erweitern und/oder Offenhalten, um so die Passierbarkeit für Spermien und/oder befruchtete Eizellen wieder herzustellen oder auf sichere Weise zu ermöglichen. Auf diese Weise ist die Transportfunktion des Eileiters gegeben und eine natürliche Befruchtung wird ermöglicht.

Gemäß einer speziellen Ausführungsform der Erfindung ist vorgesehen, dass sich die Steifigkeit des Stützelementes in Längsrichtung des Stützelementes derart kontinuierlich verändert, dass die Steifigkeit in implantiertem Zustand des Stützelementes in distaler Richtung des Eileiters abnimmt. Gemäß dieser Ausführungsform lässt sich die Steifigkeit des Stützelements an die Kontur des Eileiters anpassen. Ein wesentlicher Aspekt der Erfindung in diesem Zusammenhang ist, dass sich das Implantat, insbesondere das rohrförmige Stützelement, aufgrund des spezifischen Designs der Außenwand mit seinen Öffnungen bzw. Poren an den Verlauf des Eileiters anpasst und diesen nicht einfach begradigt. Das Stützelement mit seiner Außenwand schmiegt sich nach erfolgter Implantation vielmehr an die Eileiterwand an. Das erfindungsgemäße Implantat zum Offenhalten des Eileiters mit seinem offenporigen Stützelement passt sich somit einerseits bedarfsgerecht an die individuell geformte Eileiterwand an und andererseits hält es den benötigten Hohlkanal innerhalb des Eileiters offen. Außerdem wachsen oder ragen die Falten der Tubenschleimhaut nach Implantation des Stützelementes durch die Öffnungen der Außenwand in das Innenvolumen hinein, wodurch insbesondere der Transport einer befruchteten Eizelle begünstigt wird.

Vorzugsweise verfügt das Stützelement über eine Länge von 10 bis 20 mm und/oder die Außenwand verfügt bevorzugt über eine Dicke bzw. Wandstärke von 50 bis 100 µm.

Im Weiteren ist es von Vorteil, wenn die Außenwand des Stützelements ein gewebeverträgliches, biokompatibles und/oder degradierbares Material aufweist. Die Verwendung eines Stützelemente aus einem derart gewebeverträglichen, biokompatiblen und/oder degradierbaren Material, das aufgrund seiner radialen Steifigkeit sicherstellt, dass das Stützelement derart im Isthmus verankert ist, dass dieser dauerhaft oder über eine gewünschte Zeitspanne dilatiert ist, stellt sicher, dass das implantierte Implantat gut vertragen, insbesondere vom Körper der Patientin nicht abgestoßen wird. Wesentlich für die Auswahl eines geeigneten Materials ist es, dass diese gewebeverträglich ist. Gemäß einer besonderen Ausführungsform weist das Stützelement ein Poly(L-Lactid) auf.

Bei Verwendung eines degradierbaren, insbesondere biodegradierbaren Materials wird ferner erreicht, dass kein dauerhafter Eingriff in den Körper der behandelten Person erfolgt, sondern sich das Implantat nach einer gewünschten zersetzt und schließlich im Körper nicht mehr vorhanden ist.

In diesem Zusammenhang ist es auf vorteilhafte Weise denkbar, dass sich das Implantat, insbesondere dessen Stützelement, nach einer vorgegebenen Zeitspanne, die einige Monate oder ein bis zwei Jahre, besonders bevorzugt einen Zeitraum, in dem ein Kinderwunsch vorliegt, beträgt, im Körper verbleibt und sich dann selbstständig auflöst, etwa indem das Material hydrolytisch zersetzt, vom Körper aufgenommen oder schließlich wieder ausgeschieden wird, ohne dass ein weiterer Eingriff hierfür erforderlich ist. Ebenso ist es allerdings denkbar, dass das Implantat, insbesondere das Stützelement ein Metall, bevorzugt Magnesium, Titan und/oder eine Nickel-Titan-Legierung, aufweist. Titan bzw. eine Nickel-Titan-Legierung bieten hierbei den Vorteil, dass es im Körper gut verträglich ist und darüber hinaus ein Implantat realisierbar ist, dass auch möglicherweise im Eileiter auftretende vergleichsweise hohe Radialkräfte aufnehmen kann.

In einer besonderen Ausführungsform der Erfindungen haben die Oberflächen der Öffnungen in der Außenwand des Stützelementes einen Anteil von 70 bis 80%, insbesondere von 72 bis 77% und besonders bevorzugt von 75% an einer Außenfläche der Außenwand. Aufgrund eines derart offenporigen Designs bzw. einer Außenwand mit einer Vielzahl von Öffnungen ist gewährleistet, dass die Funktionalität des Eileiters erhalten bleibt und das Stützelement eine besondere Flexibilität aufweist und sich entsprechend genau an die Form des Eileiters anpasst. Da das Implantat aufgrund dieser besonderen Ausführungsform nur eine Oberfläche von 20 bis 30%, insbesondere von 28 bis23% und besonders bevorzugt von 25% der Oberfläche des Eileiters in dem Bereich, in dem das Implantat mit ihrem Stützelement angeordnet ist, abdeckt, wird die Funktionalität des Eileiters, insbesondere die Funktionalität der Tubenschleimhaut, die durch die Öffnungen der Außenwand hindurchwachsen und/oder -ragen kann, nur wenig oder gar nicht eingeschränkt. Im Weiteren ist es denkbar, dass durch zusätzliche Strukturierung der Oberfläche der Außenwand des Stützelements, insbesondere der dem Innenvolumen zugewandten Oberfläche der Außenwand, die Oberfläche für die Zellbesiedlung optimiert wird. Auf diese Weise können insbesondere Kinozilien auf der Oberfläche der Außenwand, in Bereichen, die dem Innenvolumen des Stützelements zugewandt sind, ausdifferenzieren und etwa Entzündungen auf vorteilhafte Weise vermieden werden. Auf diese Weise lässt sich erreichen, dass Kinozilien die dem Innenvolumen des Stützelements zugewandte Oberfläche der Außenwand überwachsen und so diesen Bereich der Außenwand zumindest bereichsweise, vorzugsweise vollständig bedecken.

Von besonderem Vorteil ist es, wenn die Außenwand auf ihrer Oberfläche, insbesondere der dem Innenvolumen zugewandten Oberfläche derart strukturiert oder mikrostrukturiert ist, dass die Außenwand Vertiefungen und/oder Aussparrungen aufweist, wodurch die Regeneration von Flimmerepithelzellen sowie die Zellbesiedlung des Implantates und damit die Transportfunktion des Eileiters unterstützt werden. In einer besonderen Weiterbildung ist vorgesehen, dass die die Aussparrungen und/oder Vertiefungen in der dem Innenvolumen zugewandten Oberfläche der Außenwand entweder geradlinig oder spiralförmig, insbesondere in Bezug auf eine Längsachse des Stützelements verlaufen.

Eine weitere spezielle Weiterbildung der Erfindung sieht vor, dass gegenüberliegende Enden der Außenwand, die die Außenwand in Längsrichtung des Stützelements begrenzen, wenigstens bereichsweise abgerundet sind. Es handelt sich hierbei um die gegenüberliegenden Enden der Außenwand des Stützelementen. Das erfindungsgemäße Stützelement verfügt im Inneren über einen Hohlkanal und ist an seinen beiden gegenüberliegenden Enden geöffnet, so dass diese jeweils einen Ein- bzw. Auslass bilden. Vorzugsweise werden die Kanten der an den gegenüberliegenden Enden vorgesehenen Öffnungen der Außenwand, die jeweils einen Ein- oder Auslass bilden, derart oberflächenbehandelt, dass in diesen Bereichen keine scharfen Kanten vorhanden sind. Bevorzugt sind die Kanten der beiden Öffnungen fein poliert und abgerundet.

Ferner ist es gemäß einer weiteren Ausgestaltung der Erfindung denkbar, dass sich eine Wandstärke der Außenwand in Längsrichtung des Stützelements verändert, bevorzugt kontinuierlich verändert. In einer derartigen Ausführungsform der Erfindung ist somit vorgesehen, dass sich die Wandstärke der Außenwand in Längsrichtung des Stützelementes verändert, wobei sich vorzugsweise der Innendurchmesser des von der Außenwand umschlossenen Innenvolumens in Längsrichtung verändert, insbesondere derart, dass nach Implantation des Stützelementes der durchschnittliche Innendurchmesser in distaler Richtung des Eileiters kleiner wird. Gemäß einer ganz besonderen Ausführungsform ist in diesem Zusammenhang vorgesehen, dass die Wandstärke an einem Ende der Außenwand (18) einen 1,1- bis 2-fachen, insbesondere einen 1,5- bis 2-fachen Wert im Vergleich zu der Wandstärke am in Längsrichtung des Stützelements (17) gegenüberliegenden Ende annimmt.

Gemäß einer weiteren Ausführungsform verändert sich ein Querschnitt des Innenvolumens des Stützelementes in Längsrichtung des Stützelements. Hierbei sind generell unterschiedliche, jeweils an die Anatomie einer Patientin und/oder der Form, Größe und Kontur eines Eileiters angepasste Veränderungen des Querschnitts des Innenvolumens denkbar. Von besonderem Vorteil ist es, wenn das Innenvolumen des Stützelementes trichterförmig, beispielsweise in Form eines geraden oder zumindest abschnittsweise gekrümmten Kegel- oder Pyramidenstumpfes ausgebildet ist. Das entsprechend ausgeführte Stützelement lässt sich auf vorteilhafte Weise derart implantieren, dass der Bereich mit dem geringeren Querschnitt des Innenvolumens im distalen Bereich des Eileiters angeordnet ist.

Eine weitere Ausführungsform der Erfindung sieht vor, dass die Außenwand auf der dem Innenvolumen zugewandten und/oder abgewandten Seite zumindest abschnittsweise mit einem Medium beschichtet ist, das ein Zellwachstum begünstigt. Vorzugsweise ist die Beschichtung derart ausgeführt, dass diese Beschichtung die Besiedlung der beschichteten Oberfläche mit Kinozilien begünstigt. Alternativ oder in Ergänzung ist es denkbar, dass die Außenwand auf der dem Innenvolumen zugewandten und/oder abgewandten Seite zumindest abschnittsweise eine Oberflächenstruktur aufweist, die ein Zellwachstum begünstigt. In diesem Zusammenhang ist es denkbar, dass zumindest ausgewählte Oberflächenbereiche mit einem geeigneten mechanischen Verfahren, beispielsweise Polieren oder Schleifen bearbeitet werden, um der Oberfläche eine besonders geeignete Struktur zu verleihen. Auch hier besteht wiederum ein Hauptziel darin, die Besiedelung der entsprechend strukturierten Oberfläche mit Kinozilien zu fördern, nachdem das Implantat in den Eileiter einer Patientin implantiert wurde.

Eine weitere spezielle Ausgestaltung der Erfindung zeichnet sich dadurch aus, dass die Außenwand des Stützelementes wenigstens abschnittsweise, und zwar auf einer dem Innenvolumen zugewandten oder abgewandten Seite, mit einem Hormon, einem Enzym, einem Vitamin und/oder einem Protein beschichtet und/oder imprägniert ist. Gemäß diesem Ausführungsbeispiel ist vorgesehen, dass die Außenwand entweder durch Beschichten der Oberfläche oder durch Imprägnieren des die Außenwand bildenden Materials mit einem entsprechenden Stoff versehen ist. Alternativ oder ergänzend ist vorgesehen, dass die Außenwand des Stützelements wenigstens abschnittsweise mit einem wachstumsfördernden, antiinfektiven, antimykotischen, Spermatozoen-Antikörper, antibiotischen, antimikrobiellen, anticanceroiden, zytostatischen und/oder einem sonstigen pharmakologischen Wirkstoff, der bedarfsgerecht ausgewählt und in die Außenwand oder auf diese aufgebracht werden kann, beschichtet und/oder imprägniert ist.

Gemäß einer ganz besonderen Ausführungsform ist vorgesehen, dass die Außenwand mit einer Drug-Eluting-Beschichtung versehen ist, die Entzündungsreaktionen über antibiotische Wirkstoffe (z.B. durch die Inkorporation von Makroliden, β-Laktamantibiotika, Cephalosporine, Linkosamine) verhindert oder durch angekoppelte Zytokine (z.B. Interleukin-4, Interleukin-10) antiinflammatorische Eigenschaften vermittelt. Weiterhin ist es denkbar, eine derartige Drug-Eluting-Beschichtung zu nutzen, um über die Hemmung spezifischer Signalkaskaden (z.B. TGFβ-Kaskade) die Proliferation der Fibroblasten und glatten Muskelzellen des Myometriums zu inhibieren und somit sklerotische Prozesse zu vermeiden. Ferner ist die Modulation von Signalkaskaden (z.B. EGF-oder WNT-Kaskade) über Inhibitoren oder durch Zytokine geeignet, um eine Besiedlung der Oberfläche der Außenwand, insbesondere auf einer dem Innenvolumen zugewandten Oberfläche der Außenwand, mit Epithelzellen zu beschleunigen. Die Freisetzung von in das Implantat inkorporierten Hormonen, wie etwa Östrogen oder Progesteron oder deren Derivate bietet ferner den Vorteil, dass im Endometrium eine schnellere Regeneration des Epithels oder über an das Implantat gekoppelte Differenzierungs- bzw. Transkriptionsfaktoren ein vermehrtes Zilienwachstum mit physiologischen Eigenschaften in den Flimmerepithelzellen des Endometriums begünstigt werden kann. Für die Beschichtung der Außenwand generell geeignete und bedarfsgerecht auswählbare Stoffe sind bspw. Geminin, Multicilin und/oder Forkhead box protein J1 (FOXJ1).

Das erfindungsgemäße Stützelement lässt sich so bedarfsgerecht für unterschiedliche Indikationen verwenden. So ist es beispielsweise denkbar, dass die Außenwand des Stützelementes mit wenigstens einem pharmakologischen Wirkstoff versehen ist, der zur Behandlung einer Entzündung und/oder einer Krankheit geeignet ist und/oder der gesundheitsfördernde Eigenschaften hat. Ebenso ist es denkbar, das Stützelement derart auszuführen, dass dieses auch der Empfängnisverhütung dient, bspw. indem dieses mit einem antispermatozoiden Medium, insbesondere mit Spermatozoen-Antikörpern, beschichtet und/oder imprägniert ist.

Eine besonders bevorzugte Ausführungsvariante sieht in diesem Zusammenhang vor, dass das Stützelement ein Depot aufweist, dass nach Implantation über einen längeren Zeitraum wenigstens einen Stoff, insbesondere einen der zuvor genannten Stoffe bzw. Wirkstoffe in eine Umgebung abgibt. In diesem Zusammenhang ist es denkbar, dass der entsprechende Stoff aus dem Depot über einen längeren Zeitraum, insbesondere mehrere Wochen oder Monate abgegeben wird, sodass über einen längeren Zeitraum ein Wirkstoff in einem hierfür vorgesehenen Zielhorizont wirken kann.

Eine ganz besonders spezielle Ausführungsform der Erfindung sieht vor, dass das Stützelement eine Membran aufweist, die einen durch das Innenvolumen gebildeten Kanal in Längsrichtung des Stützelements an einer Stelle bzw. in einem Bereich verschließt. Vorzugsweise handelt es sich bei einer derartigen Membran um ein faltbares Diaphragma.

In diesem Zusammenhang ist es denkbar, dass ein gemäß dieser Ausführungsform gestaltetes Implantat mit der im Innenvolumen angeordneten Membran in den Eileiter einer Frau implantiert und zu einem späteren Zeitpunkt, zu dem dies medizinisch möglich und/oder gewollt ist, die Membran entfernt und/oder zerstört wird, also der durch das Innenvolumen in Längsrichtung des Stützelements gebildete Kanal wieder freigegeben wird. Das Öffnen des Kanals erfolgt bevorzugt mit Hilfe eines geeigneten Kathetersystems, das in den Eileiter eingeführt wird. Mit Hilfe eines Stützelements, das die zuvor beschriebene Membran in seinem Innenvolumen aufweist, ist eine zeitweise Verschließung des Eileiters und ein gezieltes Öffnen zu einem gewünschten Zeitpunkt möglich. In diesem Zusammenhang ist es denkbar, dass der zeitweise Verschluss mit Hilfe der Membran aufgrund medizinischer Gründe erforderlich ist, beispielsweise um ein sicheres Ausheilen des Eileiters nach einer Erkrankung, Entzündung oder einem medizinischen Eingriff und/oder ein ausreichendes Einwachsen von Zellen in das Innenvolumen des Stützelements zu gewährleisten. Alternativ oder in Ergänzung ist es denkbar, dass ein Stützelement mit einer derartigen Membran für einen gewissen Zeitraum der Empfängnisverhütung dient

Im Folgenden wird die Erfindung ohne Beschränkung des allgemeinen Erfindungsgedankens anhand von Ausführungsbeispielen unter Bezugnahme auf Figuren näher erläutert. Dabei zeigen:
- Fig. 1:: Schematische Übersichtsdarstellung eines Gebärmuttertrakts mit einem Katheter und einem im Eileiter angeordneten, erfindungsgemäß ausgeführten Implantat;
- Fig. 2:: schematische Darstellung verschiedener Varianten der Formgebung für ein erfindungsgemäß ausgeführtes Implantat:
- Fig. 3:: Draufsicht und Schnittdarstellung eines erfindungsgemäß ausgeführten Implantats;
- Fig. 4:: Detaildarstellung des in Fig. 3 gezeigten Implantats;
- Fig. 5:: vergrößerte Darstellung eines Ausschnitts der in Fig. 4 gezeigten Detaildarstellung;
- Fig. 6:: schematische Darstellung eines im Eileiter angeordneten Implantats mit Stützelement
- Fig. 7:: schematische Darstellung des Transportvorgangs einer Eizelle bei unterschiedlicher Ausführung des erfindungsgemäßen Implantats;
- Fig. 8:: Darstellung mehrerer Verfahrensschritte zur Implantation des erfindungsgemäßen Implantats in einen Eileiter

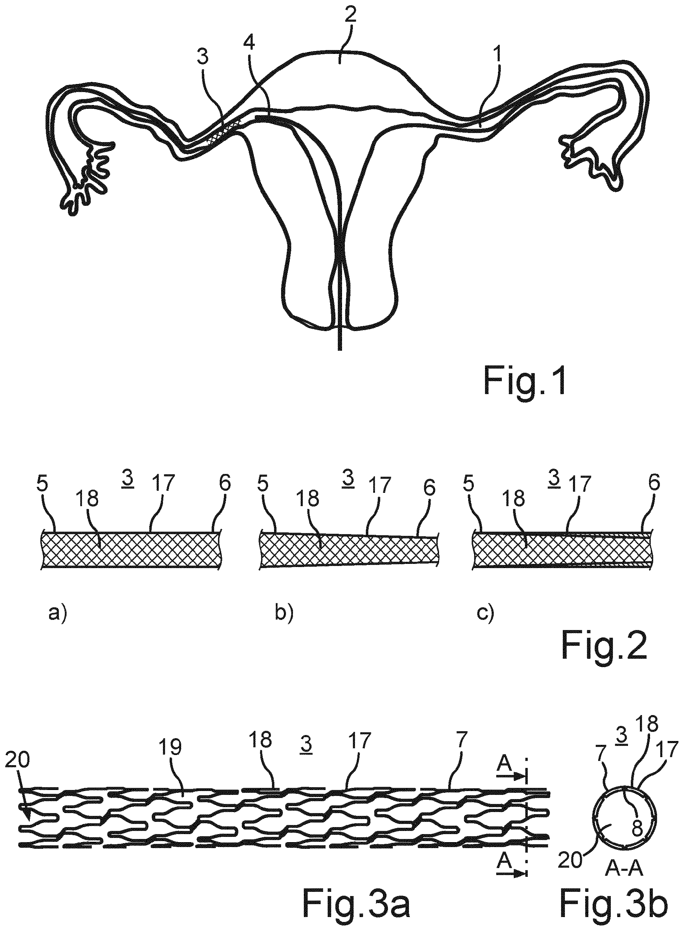

Fig. 1 zeigt in einer schematischen Darstellung den weiblichen Geschlechtstrakt, wobei in der hier gewählten Ansicht dargestellt lediglich die für die Erfindung relevanten Teile, insbesondere die Gebärmutter 2 und der Eileiter 1 gezeigt sind. Weiterhin ist schematisch eine mögliche Positionierung eines Implantats 3 mit einem Stützelement 17 sowie ein für die Implantation geeignetes Applikationssystem 4 gezeigt.

In Ergänzung sind in Fig. 2 unterschiedliche Varianten einer Gestaltung eines erfindungsgemäßen Implantats 3 mit einem Stützelement 17 gezeigt, wobei in Fig. 2a) eine erste, in Fig. 2b) eine zweite sowie in Fig. 2c) eine dritte Variante einer möglichen Form des Stützelements 17 mit seiner Außenwand 18 dargestellt ist.

Das erfindungsgemäße Implantat 3 verfügt über ein flexibles, rohrförmiges, beispielsweise zylindrisches Stützelement 17, das eine Außenwand 18 aufweist, welche ein Innenvolumen 19 des Stützelements 17 umfangsseitig umgibt. Das Stützelement 17 ist somit hohl und verfügt über ein erstes Ende 5, das nach erfolgter Implantation proximal im Eileiter angeordnet ist, und ein zweites Ende 6, das nach erfolgter Implantation distal im Eileiter angeordnet ist. Während die Außenwand 18 gemäß der in Fig. 2a) ge- zeigten ersten Variante eine in Längsrichtung des Stützelements 17 konstante Wand- stärke aufweist und zylindrisch geformt ist, zeigt Fig 2b) eine zweite Variante des erfindungsgemäßen Implantats, bei der die Außenwand 18 des Stützelements 17 eine kon- stante Wandstärke aufweist und das Stützelement 17 nicht zylindrisch geformt ist. Weiterhin ist in Fig. 2c) eine dritte Ausführungsform des erfindungsgemäßen Implan- tats 3 gezeigt, bei der das Stützelement 17 nicht zylindrisch geformt ist und die Wand- stärke der Außenwand 18 in Längsrichtung des Stützelementes 17 nicht konstant ist, sich also in Längsrichtung des Stützelements 17 verändert.

In Fig. 3 wird eine erste Ausführungsform des erfindungsgemäßen Implantats 3 dargestellt, wobei in Fig. 3a) eine Draufsicht auf das Implantat 3 und in Fig. 3b) eine Darstellung des Schnittes A-A durch das Implantat 3 gezeigt ist. Das Implantat verfügt über ein Stützelement 17, das eine Außenwand 18 mit einer Vielzahl von Öffnungen 19 aufweist. Gemäß der in Fig. 3 gezeigten Ausführungsform weist die Außenwand 18 eine netzartige Gestalt auf. Diese netzartige Außenwand 18 wird durch einzelne Struts 7 gebildet, die gemäß der in Fig. 3 gezeigten Ausführungsform meanderförmig ausgebildet sind. Weiterhin ist die dem Innenvolumen zugewandte Oberfläche der Außenwand derart strukturiert bzw. mikrostrukturiert, dass die Struts über Nuten 8 verfügen, die nach erfolgter Implantation den Eizellentransport und die Besiedlung mit Kinozilien 10 unterstützen.

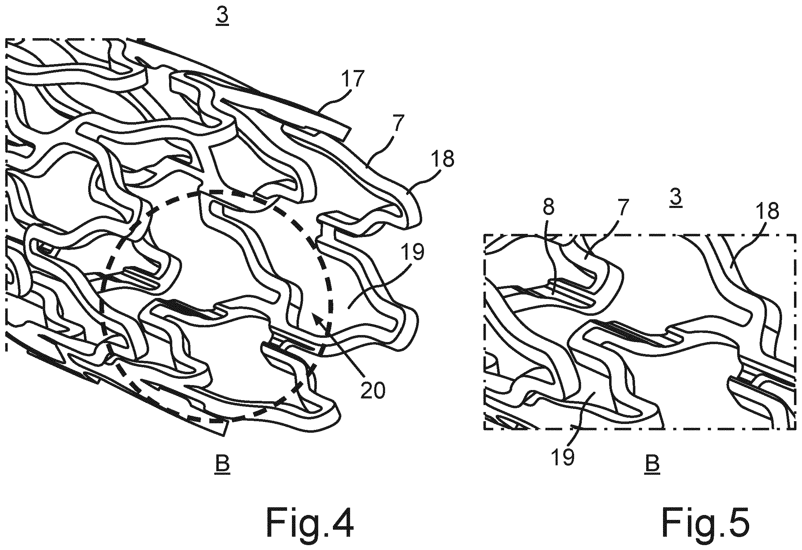

In Ergänzung zu Fig. 3 zeigt Fig. 4 eine dreidimensionale Detaildarstellung einer Ausführungsform des erfindungsgemäßen Implantats. In dieser Ansicht ist insbesondere die Gestaltung der Außenwand 18 des Stützelements 17, die gemäß der gezeigten Ausführungsform netzartig ausgebildet ist, wobei die Öffnungen 19 einen besonders großen Flächenanteil ausmachen, zu erkennen. Weiterhin zeigt Fig. 5 in einer weiteren Detaildarstellung einen nochmals vergrößerten Ausschnitt der Außenwand 18, wobei deutlich die Oberflächenstruktur in Form von Nuten 8 zu sehen ist.

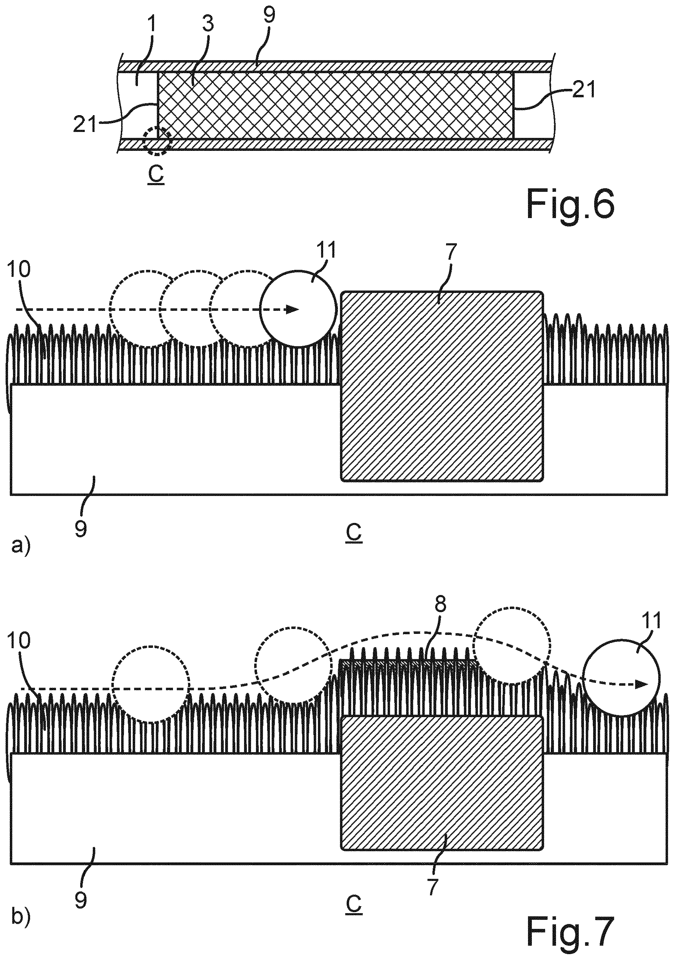

In Fig. 6 ist ferner eine schematische Darstellung eines Implantats 3 nach erfolgter Implantation in einen Eileiter 1 dargestellt. Das Implantat befindet sich innerhalb des Eileiters 1 und die äußere Oberfläche der Außenwand 18 liegt dicht an der Eileiterwand 9 an. Aufgrund der Vielzahl von Öffnungen 19 in der Außenwand und der speziellen Gestaltung der Außenwand 18 ist es nun möglich, dass Falten einer Schleimhaut des Eileiters 1 durch die Öffnungen 19 in das Innenvolumen 20 hindurchwachsen

In Ergänzung zu der schematischen Darstellung in Fig. 6 zeigen die Figuren 7a) un 7b) Detaildarstellung des Bereiches C am distalen Ende des Implantats, und zwar in einem Zustand, in dem sich eine Eizelle 11 im Eileiter befindet. Die Kanten 21 an diesem ersten Ende 5 des Stützelement sind abgerundet und feinst poliert, sodass am Übergang von dem durch das Implantat offengehaltenen Gewebebereich des Eileiters und dem Gewebebereich des Eileiters, in den sich das Implantat nicht befindet, ein Übergangswinkel, der kleiner als 90° ist, realisiert wird.

Das in Fig. 7a) gezeigte Implantat 1 verfügt über ein Stützelement 17 mit einer Außenwand 18, deren Öffnungen 19 zwischen Struts 7 angeordnet sind, wobei gemäß der in Fig. 7a) gezeigten Ausführungsform auf der der Eileiterwand 9 gegenüberliegenden Oberfläche der Struts 7 keine Struktur in Form von Nuten 8 vorgesehen ist. Wie Fig. 7a) verfängt sich die Eizelle 11 an dem Strut 7 ohne Oberflächenstrukturierung, da die Kinozilien 10 nur auf der Eileiterwand 9 ausdifferenziert sind.

Demgegenüber ist Fig. 7b) ein erfolgreicher Transport einer Eizelle 11 durch den von einem erfindungsgemäßen Implantat offengehaltenen Eileiter zu erkennen. Aufgrund der Strukturierung der dem Innenvolumen zugewandten Oberfläche der Struts 7, die die Außenwand 18 des Stützelements 17 bilden, mit einer Nut 8 wird die Ansiedlung von Kinozilien begünstigt, die sich bereits nach vergleichsweise kurzer Zeit nach erfolgter Implantation auf der Oberfläche der Struts 7 ausdifferenzieren können.

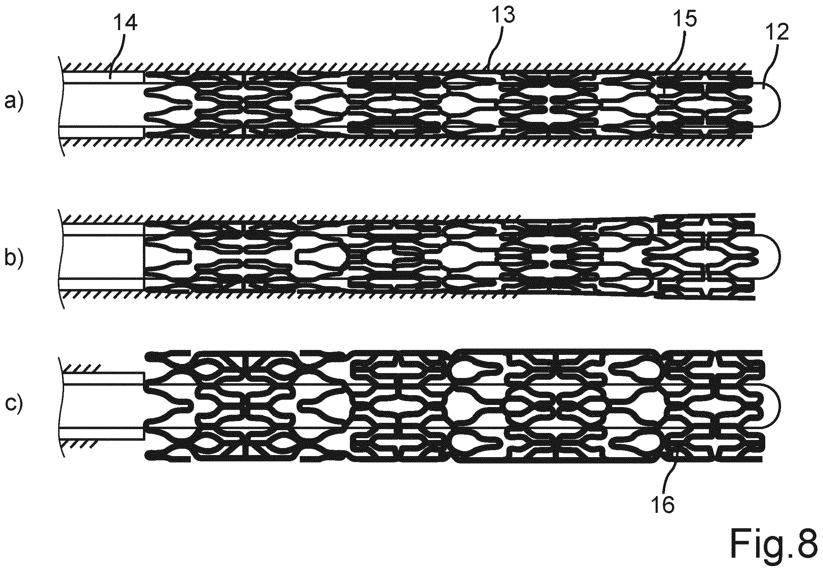

Abschließend zeigt Fig. 8 zur Verdeutlichung einer möglichen Verwendung des erfindungsgemäßen Implantats 3 den Ablauf eines geeigneten minimalinvasiven Implantationsverfahren. In diesem Zusammenhang zeigen die Figuren 8a), 8b) und 8c) schematisch die verschiedenen, während der Implantation durchgeführten Verfahrensschritte. In Fig. 8a) ist das mit einem Implantat 3 geladene Applikationssystem 4 gezeigt, das über einen Führungsdraht 12, einen äußeren Schaft 13 und einem Hohlstempel 14 verfügt. Fig. 8b) zeigt einen Verfahrensschritt, bei dem das teilweise noch gecrimpte Implantat 15, das sich hierbei in einem zusammengedrückten Zustand innerhalb des äußeren Schafts 13 befindet, langsam und kontrolliert durch das Zurückziehen des äußeren Schafts 13 freigesetzt wird, sodass sich das Implantat 3 in entfaltetem Zustand an die Eileiterwand 9 anlegt. Während dieses Verfahrensschrittes fixiert der Hohlstempel 14 das Implantat 3, 15 an der Implantationsstelle. Schließlich ist in Fig. 8c) das vollständig im Eileiter 3 freigesetzte Implantat 3 dargestellt.

### Bezugszeichenliste

- 1: Eileiter
- 2: Gebärmutter
- 3: Implantat
- 4: Applikationssystem
- 5: erstes Ende
- 6: zweites Ende
- 7: Strut
- 8: Nut
- 9: Eileiterwand
- 10: Kinozilien
- 11: Eizelle
- 12: Führungsdraht
- 13: äußerer Schaft
- 14: Hohlstempel
- 15: Gecrimpter Stent
- 16: Expandierter Stent
- 17: Stützelement
- 18: Außenwand
- 19: Öffnungen
- 20: Innenvolumen
- 21: endseitige Kanten des Stützelements

## Patentansprüche

1. Implantat (3) zur Wiederherstellung der Durchgängigkeit und Funktionalität eines Eileiters (1) im Reproduktionsgeschehen in Längsrichtung des Eileiters (1), das über ein Stützelement (17) mit einer rohrförmigen, ein Innenvolumen (20) des Stützelements (17) zumindest teilweise begrenzenden und einen zumindest annähernd kreisförmigen Querschnitt aufweisenden Außenwand (18) verfügt, wobei in der Außenwand (18) Öffnungen (19) vorgesehen sind, die derart ausgebildet sind, dass zumindest zeitweise nach Implantation des Stützelements (17) Falten einer Schleimhaut des Eileiters (1) wenigstens bereichsweise durch die Öffnungen (19) in das Innenvolumen (20) hindurchwachsen und/oder hindurchragen, **dadurch, gekennzeichnet, dass** eine Steifigkeit des Stützelements (17) in Längsrichtung des Stützelements variiert.

2. Implantat nach Anspruch 1,
**dadurch gekennzeichnet, dass** das sich die Steifigkeit des Stützelements (17) in Längsrichtung des Stützelements (17) derart kontinuierlich verändert, dass die Steifigkeit in implantiertem Zustand des Stützelements (17) in distaler Richtung des Eileiters (1) abnimmt.

3. Implantat nach Anspruch 1 oder 2,
**dadurch gekennzeichnet, dass** das Stützelement (17) in Längsrichtung eine Länge von 10 bis 20 mm aufweist.

4. Implantat nach einem der vorangehenden Ansprüche,
**dadurch gekennzeichnet, dass** die Außenwand (18) des Stützelements (17) einen Außendurchmesser von 0,8 bis 2,5 mm aufweist und/oder über ein gewebeverträgliches und/oder biodegradierbares Material verfügt.

5. Implantat nach einem der vorangehenden Ansprüche,
**dadurch gekennzeichnet, dass** Oberflächen der Öffnungen (19) einen Anteil von 70 bis 80%, insbesondere von 72 bis 77% und besonders bevorzugt von 75% an einer Außenfläche der Außenwand (18) haben.

6. Implantat nach einem der vorangehenden Ansprüche,
**dadurch gekennzeichnet, dass** die Außenwand (18) des Stützelements (17) eine Wandstärke von 50 bis 100 µm aufweist und/oder Enden der Außenwand (18), die die Außenwand (18) in Längsrichtung begrenzen, wenigstens bereichsweise abgerundet sind.

7. Implantat nach einem der vorangehenden Ansprüche,
**dadurch gekennzeichnet, dass** sich eine Wandstärke der Außenwand (18) in Längsrichtung verändert.

8. Implantat nach Anspruch 7,
**dadurch gekennzeichnet, dass** die Wandstärke an einem Ende der Außenwand (18) einen 1,1- bis 2-fachen, insbesondere einen 1,5- bis 2-fachen Wert im Vergleich zu der Wandstärke am in Längsrichtung des Stützelements (17) gegenüberliegenden Ende annimmt.

9. Implantat nach einem der vorangehenden Ansprüche,
**dadurch gekennzeichnet, dass** die Außenwand (18) auf der dem Innenvolumen (20) zugewandten und/oder abgewandten Seite zumindest abschnittsweise mit einem Medium beschichtet ist, das ein Zellwachstum begünstigt.

10. Implantat nach einem der vorangehenden Ansprüche,
**dadurch gekennzeichnet, dass** die Außenwand (18) auf der dem Innenvolumen zugewandten und/oder abgewandten Seite zumindest abschnittsweise eine Oberflächenstruktur, insbesondere wenigstens eine Nut (8), aufweist, die ein Zellwachstum begünstigt.

11. Implantat nach Anspruch 10,
**dadurch gekennzeichnet, dass** die wenigstens eine Nut (8) parallel und/oder spiralförmig zur Längsmittenachse des Innenvolumens (20) des Stützelements (17) angeordnet ist.

12. Implantat nach einem der vorangehenden Ansprüche,
**dadurch gekennzeichnet, dass** die Außenwand (18) des Stützelements (17) ein Metall und/oderein Poly-L-Lactid aufweist.

13. Implantat nach einem der vorangehenden Ansprüche,
**dadurch gekennzeichnet, dass** sich ein Querschnitt des Innenvolumens (20) des Stützelements (17) in Längsrichtung verändert.

14. Implantat nach einem der vorangehenden Ansprüche,
**dadurch gekennzeichnet, dass** das Innenvolumen (20) das Stützelements (17) trichterförmig und/oder in Form eines geraden oder zumindest abschnittsweise gekrümmten Kegelstumpfs oder Pyramidenstumpfs ausgebildet ist.

15. Implantat nach einem der vorangehenden Ansprüche,
**dadurch gekennzeichnet, dass** die Außenwand (18) des Stützelements (17) wenigstens abschnittsweise mit einem Hormon, einem Enzym, einem Vitamin und/oder einem Protein beschichtet und/oder imprägniert ist.

16. Implantat nach einem der vorangehenden Ansprüche,
**dadurch gekennzeichnet, dass** die Außenwand (18) des Stützelements (17) wenigstens abschnittsweise mit einem wachstumsfördernden, antiinfektiven, antimykotischen, antispermatozoiden, antibiotischen, antimikrobiellen, anticanceroiden und/oder einem sonstigen pharmakologischen Wirkstoff beschichtet und/oder imprägniert ist.

17. Implantat nach einem der vorangehenden Ansprüche,
**dadurch gekennzeichnet, dass** das Stützelement (17) ein Depot aufweist, das nach Implantation über einen längeren Zeitraum wenigstens einen Stoff in eine Umgebung abgibt.

18. Implantat nach einem der vorangehenden Ansprüche,
**dadurch gekennzeichnet, dass** das Stützelement (17) eine das Innenvolumen über dessen Querschnitt verschließende, einmalig zu öffnende Membran aufweist.
